# EUROPEAN PATENT APPLICATION

(11) **EP 3 919 827 A1**
(43) Date of publication of application: **08.12.2021**
(21) Application number: 21177876.6
(22) Date of filing: 04.06.2021
(51) Int. Cl.: F24F 8/167, A61L 9/20

(54) **UV SYSTEMS FOR PATHOGEN MITIGATION IN HVAC**

(30) Priority: 04.06.2020 US 202063034588 P
(71) Applicant: Koninklijke Fabriek Inventum B.V., 3439 MC Nieuwegein (NL)
(72) Inventor: Vermeulen, Timothy, Nieuwegein (NL)
(74) Representative: Dehns

(57) **Abstract**

A system includes an air duct and an ultra-violet (UV) photocatalysis system in fluid communication with the air duct for reduction of airborne pathogens in air flow through the air duct. An HVAC system can is fluid communication with the air duct, where the HVAC system is upstream and/or downstream of the air duct. The UV photocatalysis system can include at least one vacuum UV (VUV) illuminator positioned in the air duct to illuminate at least one photocatalyst body. The photocatalyst body can be configured to catalyze ionization of pathogens in airflow through the air duct under illumination from the VUV illuminator.

## Description

### BACKGROUND

### 1. Field

The present disclosure relates to pathogen reduction, and in particular to pathogen reduction in HVAC systems such as aboard aircraft.

### 2. Description of Related Art

The COVID-19 pandemic as underscored the importance of pathogen reduction aboard aircraft. Effective destruction of pathogens onboard aircraft is an important way to reduce risk of infection to passengers. There is an ongoing need for systems and methods to improve pathogen reduction aboard aircraft, and this disclosure provides a solution for this need.

The conventional techniques have been considered satisfactory for their intended purpose. However, there is an ever present need for improved systems and methods for efficient air disinfection. This disclosure provides a solution for this need.

### SUMMARY

A system includes an air duct and an ultra-violet (UV) photocatalysis system in fluid communication with the air duct for reduction of airborne pathogens in air flow through the air duct. An HVAC system can is fluid communication with the air duct, where the HVAC system is upstream and/or downstream of the air duct. The UV photocatalysis system can include at least one vacuum UV (VUV) illuminator positioned in the air duct to illuminate at least one photocatalyst body. The photocatalyst body can be configured to catalyze ionization of pathogens in airflow through the air duct under illumination from the VUV illuminator.

In certain embodiments, the HVAC system and the UV photocatalysis system can be configured to photocatalyze airborne pathogens passing through the air duct with a residence time of 0.125 seconds or less within the UV photocatalysis system. In certain embodiments, the HVAC system can be a sub-system of an environmental control system (ECS).

In embodiments, the UV photocatalysis system can include the at least one photocatalyst body mounted in the air duct, the at least one photocatalyst body being permeable to air flow therethrough. The photocatalyst body can further include an illumination chamber.

In embodiments, the air duct, the at least one VUV illuminator, and the at least one photocatalyst can be configured to ionize pathogens passing through the UV photocatalysis system with a residence time of 0.125 seconds or less. In certain embodiments, the VUV illuminator can emit at wavelengths less than or equal to 200 nm.

In certain embodiments, a method includes catalyzing ionization of pathogens in airflow through an air duct under illumination from a vacuum UV (VUV) illuminator of an ultraviolet (UV) photocatalysis system. The method can include photocatalyzing airborne pathogens passing through the air duct with a residence time of 0.125 seconds or less within the UV photocatalysis system. In certain embodiments, the VUV illuminator can be positioned in the air duct such that the method can further include illuminating the at least one photocatalyst body. In certain embodiments, the method can include ionizing pathogens passing through the UV photocatalysis system with a residence time of 0.125 seconds or less.

In certain embodiments in accordance with at least one aspect of this disclosure, a method for neutralizing airborne pathogens in an aircraft can include retrofitting an aircraft environmental control system (ECS) with the ultra-violet (UV) photocatalysis system described above. Retrofitting can include inserting the UV photocatalysis system into an existing duct of the ECS.

These and other features of the systems and methods of the subject disclosure will become more readily apparent to those skilled in the art from the following detailed description taken in conjunction with the drawings.

### BRIEF DESCRIPTION OF THE DRAWINGS

So that those skilled in the art to which the subject disclosure appertains will readily understand how to make and use the devices and methods of the subject disclosure without undue experimentation, embodiments thereof will be described in detail herein below with reference to certain figures, wherein:

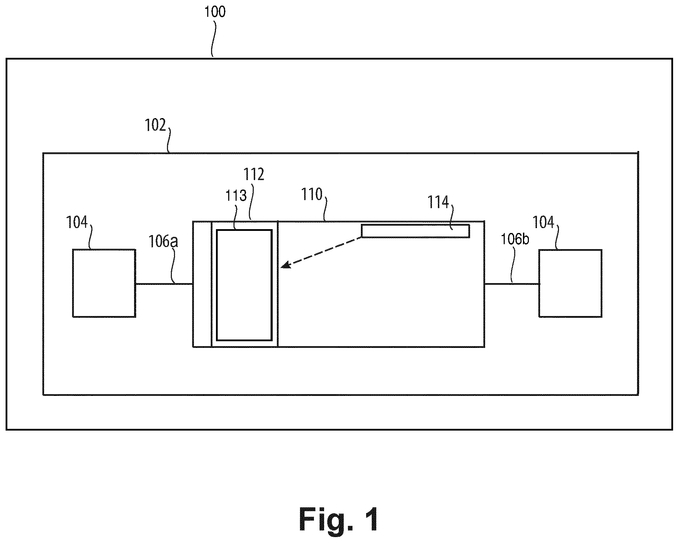

Fig. 1 is a diagrammatic view of an embodiment of an environmental control system constructed in accordance with the present disclosure, showing a neutralization system incorporated into the environmental control system.

### DETAILED DESCRIPTION

Reference will now be made to the drawings wherein like reference numerals identify similar structural features or aspects of the subject disclosure. For purposes of explanation and illustration, and not limitation, a partial view of an embodiment of a system in accordance with the disclosure is shown in Fig. 1 and is designated generally by reference character 100. The systems and methods described herein can be used to neutralize pathogens from air flow systems.

In certain embodiments, an aircraft 100 can include an environmental control system (ECS) 102, where the ECS 102 includes an HVAC subsystem 104. The HVAC system 104 is in fluid communication with an air duct 106, the HVAC system 104 being upstream and/or downstream of the air duct 106. A neutralization system 108 can include the air duct 106 and an ultra-violet (UV) photocatalysis system 110 in fluid communication with the air duct 106, for example for reduction of airborne pathogens in air flow through the air duct 106.

In certain embodiments, the HVAC system 104 and the UV photocatalysis system 110 can be configured to photocatalyze airborne pathogens passing through the air duct 106 with a residence time of 0.125 seconds or less within the UV photocatalysis system 110. At least one photocatalyst body 112 can be mounted in the air duct 106, the at least one photocatalyst body 112 being permeable to air flow therethrough. For example, the photocatalyst body 112 includes a flow path from an inlet portion 106a of the air duct 106 through an illumination chamber 113 of the photocatalyst body 112, and to an outlet portion 106b of the air duct 106.

At least one vacuum UV (VUV) illuminator 114 can be positioned in the air duct 106 to illuminate the at least one photocatalyst body 112 at the illumination chamber 113 such that the photocatalyst body 112 can catalyze ionization of pathogens in airflow through the air duct 106 under illumination from the VUV illuminator 114 (e.g. as indicated by the dashed arrow). The VUV illuminator can be a separate body as shown, configured to illuminate the illumination chamber 113 from a distance, or the VUV illuminator 114 can be integral with the photocatalyst body 112 configured to illuminate the photocatalyst body 112 from within. As used herein, the term vacuum ultraviolet refers to the short-wavelength portion of the electromagnetic spectrum where the photons are energetic enough to excite a typical atom from the ground state to ionization.

In embodiments, the air duct 106, the at least one VUV illuminator 114, and the at least one photocatalyst body 112 can be configured to ionize pathogens passing through the UV photocatalysis system 110 with a residence time of 0.125 seconds or less. An irradiating duration of <0.125 seconds provided by the VUV illuminator 114 is advantageous over conventional UV which requires longer irradiation time to successfully inactivate bacteria, viruses, and other pathogens. For example, an irradiation time of <0.125 for conventional UV results in only 50% efficiency and efficacy of inactivating certain bacteriophages, while VUV can result in 100% inactivation for those same bacteriophages. In certain embodiments, the VUV illuminator 114 can emit at wavelengths less than or equal to 200 nm, which has a higher photon energy (6.70 eV) than other UV sources (4.88 and 3.40 eV) having longer UV wavelength (254 and 365Hz).

In certain embodiments, a method can include catalyzing ionization of pathogens in airflow through the air duct 106 under illumination from a vacuum UV (VUV) illuminator 114 of an ultraviolet (UV) photocatalysis system 110. The method can include photocatalyzing airborne pathogens passing through the air duct 106 with a residence time of 0.125 seconds or less within the UV photocatalysis system 110. In certain embodiments, the VUV illuminator 114 can be positioned in the air duct 106 such that the method can further include illuminating the at least one photocatalyst body 112 (e.g. at an illumination chamber 113).

In certain embodiments in accordance with at least one aspect of this disclosure, a method for neutralizing airborne pathogens in an aircraft 100 can include retrofitting an aircraft environmental control system (ECS) 102 with the ultra-violet (UV) photocatalysis system 110 as described above. For example, the photocatalysis system 110 can be easily inserted into an existing duct in ECS 102 as a unit without significant reconstruction of the ECS 102.

The methods and systems of the present disclosure, as described above and shown in the drawings, provide for more efficient and effective air neutralizing for removing pathogens in an airflow. Because of the short irradiation time, the VUV illuminator 114 can be used in high volume airflow systems such as UV: C systems, because short irradiation times allows for higher volume airflow. Therefore, the photocatalysis system 110 can be particularly useful for high volume airflow systems such as the HVAC systems on board aircraft, for example.

While the apparatus and methods of the subject disclosure have been shown and described, those skilled in the art will readily appreciate that changes and/or modifications may be made thereto without departing from the scope of the subject disclosure.

## Claims

1. A system comprising:
an air duct;
an ultra-violet (UV) photocatalysis system in fluid communication with the air duct for reduction of airborne pathogens in air flow through the air duct; wherein the UV photocatalysis system includes at least one vacuum UV (VUV) illuminator positioned in the air duct to illuminate at least one photocatalyst body, wherein the at least one photocatalyst body is configured to catalyze ionization of pathogens in airflow through the air duct under illumination from the VUV illuminator.

2. The system as recited in claim 1, further comprising an HVAC system in fluid communication with the air duct, wherein the HVAC system is upstream and/or downstream of the air duct.

3. The system as recited in claim 2, wherein the HVAC system and the UV photocatalysis system are configured to photocatalyze airborne pathogens passing through the air duct with a residence time of 0.125 seconds or less within the UV photocatalysis system.

4. The system as recited in claim 2 or 3, further comprising an environmental control system (ECS) of an aircraft, wherein the HVAC system is a sub-system of the ECS.

5. The system as recited in any preceding claim, further comprising the photocatalyst body, the at least one photocatalyst body mounted in the air duct, wherein the at least one photocatalyst body is permeable to air flow therethrough, wherein, the photocatalyst body optionally further includes an illumination chamber.

6. The system as recited in any preceding claim, wherein the air duct, the at least one VUV illuminator, and the at least one photocatalyst are configured to ionize pathogens passing through the UV photocatalysis system with a residence time of 0.125 seconds or less.

7. The system as recited in any preceding claim, wherein the VUV illuminator emits at wavelengths less than or equal to 200 nm.

8. A method comprising:
catalyzing ionization of pathogens in airflow through an air duct under illumination from a vacuum UV (VUV) illuminator of an ultraviolet (UV) photocatalysis system.

9. The method as recited in claim 8, wherein an HVAC system in fluid communication with the air duct, wherein the HVAC system is upstream and/or downstream of the air duct, wherein the HVAC system is optionally a sub-system of an an existing environmental control system (ECS)..

10. The method as recited in claim 8 or 9, further comprising photocatalyzing airborne pathogens passing through the air duct with a residence time of 0.125 seconds or less within the UV photocatalysis system.

11. The system as recited in claim 8, 9 or 10, wherein the UV photocatalysis system includes at least one photocatalyst body mounted in the air duct, wherein the at least one photocatalyst body is permeable to air flow therethrough.

12. The system as recited in claim 11, wherein the VUV illuminator is positioned in the air duct, and further comprising illuminating the at least one photocatalyst body.

13. The system as recited in any of claims 8-12, further comprising, ionizing pathogens passing through the UV photocatalysis system with a residence time of 0.125 seconds or less.

14. A method for neutralizing airborne pathogens in an aircraft, comprising:
retrofitting an aircraft environmental control system (ECS) with the ultra-violet (UV) photocatalysis system of any preceding claim.

15. The method as recited in claim 14, wherein retrofitting further includes inserting the UV photocatalysis system into an existing duct of the ECS.
